# DEMANDE DE BREVET EUROPEEN

(11) **EP 1 034 744 A1**
(43) Date de publication de la demande: **13.09.2000**
(21) Numéro de dépôt: 00400563.3
(22) Date de dépôt: 02.03.2000
(51) Int. Cl.: A61B 17/16, A61B 17/68

(54) **Ensemble de moyens nécessaires à la réduction chirurgicale des instabilités de l'épaule**

(30) Priorité: 09.03.1999 FR 9902883
(71) Demandeur: Orthomed, 21160 Marsannay la Côte (FR)
(72) Inventeur: Doursounian, Levon, 75016 Paris (FR); Saillant, Gérard, 78170 La Celle Saint Cloud (FR)
(74) Mandataire: Robert, Jean-Pierre

(57) **Abrégé**

Ensemble de moyens nécessaires à la réduction chirurgicale des instabilités de l'épaule selon la méthode LATARJET, par mise en place d'un greffon, comprenant un matériel ancillaire et un matériel implantable. Le matériel ancillaire comporte trois outils à utiliser consécutivement à savoir un pointeau, une broche, et une mèche, chaque outil formant un organe de guidage pour la mise en place de l'outil suivant, le pointeau formant organe de guidage du greffon. Le matériel implantable comporte une cheville et une vis.

## Description

La présente invention concerne un ensemble de moyens (dispositifs médicaux et outillage ancillaire) pour la réalisation d'interventions chirurgicales destinées à réduire les instabilités de l'épaule.

Il existe plusieurs méthodes chirurgicales pour obtenir ce résultat et l'une d'elle, appelée intervention de LATARJET du nom du chirurgien qui l'a mise au point, consiste à réaliser une butée antéro-inférieure qui se substitue fonctionnellement au pôle antéro-inférieur de la glène. Cette butée est constituée par un prélèvement de l'apophyse coracoïde rapportée perpendiculairement ou couchée au bord antérieur de la glène.

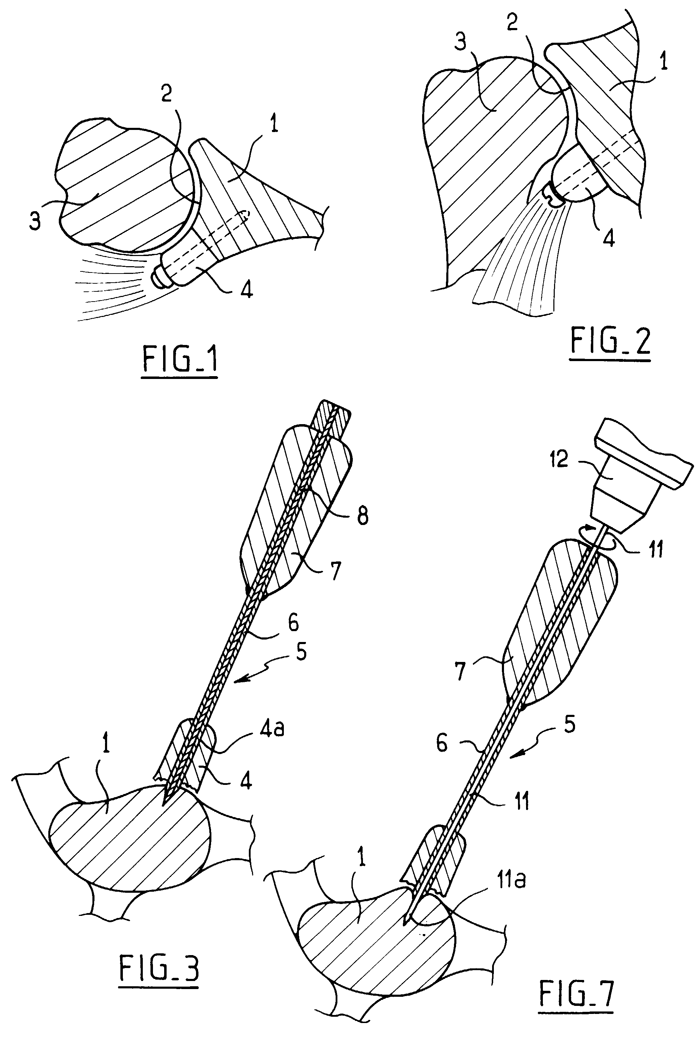

Les figures 1 et 2 illustrent par deux coupes sensiblement orthogonales les résultats d'une telle intervention. On y reconnaît l'omoplate 1 avec la glène 2 d'articulation de la tête humérale 3. La butée coracoïdienne 4 est maintenue en place par vissage sur l'omoplate dans une position très précise afin de prolonger correctement le bord antérieur de la glène et de combler le point d'échappée de la tête humérale qui constitue la luxation à réduire.

Cette opération est délicate, demande beaucoup de soin, de temps et, essentiellement manuelle, elle est sujette à l'aléa opératoire et en particulier à une imprécision de l'orientation de la butée coracoïdienne. En effet, la mise en place précise de cette butée coracoïdienne détermine la qualité de l'intervention et cette mise en place est d'exécution délicate et imprécise compte tenu de l'étroitesse du champ et des freins et liens qui limitent la mobilité et la liberté de manipulation du greffon osseux formant la butée.

Par la présente invention on fournit au chirurgien un ensemble de moyens aidant d'une part à la présentation du greffon et d'autre part, une fois la position de celui-ci arrêtée, à la conservation de cette position de manière certaine pendant toutes les opérations intermédiaires entre sa présentation et sa fixation définitive à l'omoplate. On améliore ainsi sensiblement la mise en place de la butée coracoïdienne (greffon) de laquelle dépend pour une large part le succès et la pérennité de l'invention.

A cet effet l'invention a donc pour objet un ensemble de moyens nécessaires à la réduction chirurgicale de l'instabilité de l'épaule, comprenant un matériel ancillaire et un dispositif implantable, le matériel ancillaire comportant trois outils à utiliser consécutivement, à savoir un pointeau, une broche et une mèche, chaque outil formant un organe de guidage pour la mise en place de l'outil suivant, le pointeau formant organe de guidage du greffon alors que le matériel implantable comporte une cheville et une vis.

De manière préférée, le pointeau comporte un manche solidaire d'un corps tubulaire et un clou logé de manière amovible dans le corps tubulaire dont l'extrémité libre est en forme de pointe complémentaire de l'extrémité libre du corps tubulaire et comportant des moyens de sa liaison détachable avec le corps tubulaire.

La broche quant à elle est formée d'une tige de longueur supérieure à celle du pointeau, de diamètre égal au diamètre intérieur du corps tubulaire et façonnée à l'une de ses extrémités à la manière d'une mèche. En outre, la mèche susdite est tubulaire de diamètre intérieur égal au diamètre extérieur de la broche et dont le diamètre extérieur est étagé. La longueur axiale de la section de la mèche de plus grand diamètre est déterminée par un épaulement de butée.

Enfin, dans l'ensemble selon l'invention, la cheville est une cheville à visser autotaraudeuse dont le diamètre à fond de filet est sensiblement égal au diamètre de la mèche, le diamètre au sommet de filet étant décroissant vers l'extrémité sur une section de la cheville proche de cette extrémité pour former une section d'entrée de la cheville dans l'os tandis que cette cheville comporte un orifice central interne taraudé débouchant à chacune de cette extrémité. La vis quant à elle possède sous sa tête une portion non filetée de longueur égale à celle du greffon et, à l'opposé de sa tête, une pointe pyramidale réalisée sur une partie non filetée de diamètre réduit de son extrémité.

D'autres caractéristiques et avantages de l'invention apparaîtront au cours de la description qui en est faite ci-après d'un exemple de réalisation non limitatif.

Il sera fait référence aux dessins annexés parmi lesquels, outre les figures 1 et 2 déjà décrites :
- les figures 3, 7 et 8 illustrent les différentes phases préparatoires de la mise en place du greffon osseux avec l'ancillaire adapté et,
- les figures 4, 5 et 6 sont des vues de détail d'un pointeau conforme à l'invention,
- la figure 9 est une vue en coupe d'une cheville implantée et
- la figure 10 est la vue d'une vis coopérant avec la cheville au maintien du greffon sur l'omoplate.

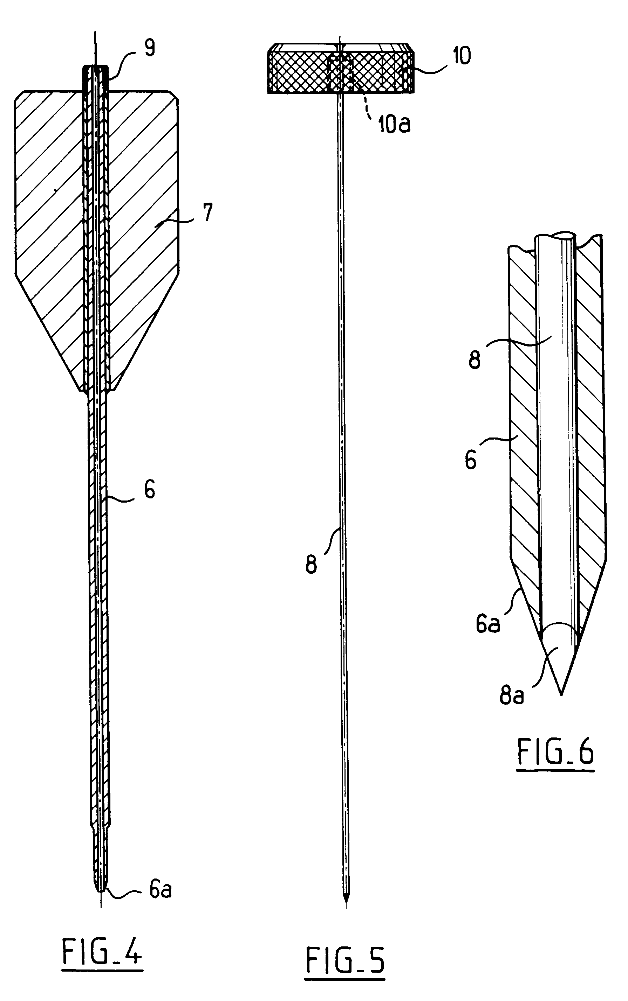

Selon la technique de LATARJET, le prélèvement de la coracoïde s'opère avant ou après avoir réalisé un orifice dans celle-ci sensiblement perpendiculaire ou parallèle au plan de sa résection. Cet orifice 4a permet au chirurgien d'y introduire un pointeau 5 formé par un fût tubulaire 6 équipé d'un manche 7 et d'un clou central 8. La figure 4 qui est une vue en coupe de la partie de pointeau formée du fût et du manche fait apparaître que le fût tubulaire 6 étant à son extrémité libre taillé en pointe d'angle au sommet de préférence égal à 40° tandis qu'à l'extrémité opposée à cette pointe le fût 6 fait saillie au-delà du manche 7 par une extrémité 9 filetée. L'alésage interne du pointeau est d'un diamètre égal au diamètre extérieur du clou 8 cylindrique représenté à la figure 5.

A l'une de ses extrémités 8a, le clou est taillé en pointe, de préférence en pointe pyramidale d'angle au sommet égal aussi à 40° (voir figure 6). A l'autre extrémité le clou 8 est soudé à un bouchon 10 en traversant un taraudage central 10a grâce auquel le bouchon 10 peut être vissé sur l'extrémité filetée 9 de la douille 7a. Lorsque l'on visse à fond le clou sur le manche, le chapeau 10 prend appui sur la surface supérieure du manche 7 et le pointeau constitue un outil qui peut être frappé pour être impacté dans l'os de l'omoplate comme l'illustre la figure 3.

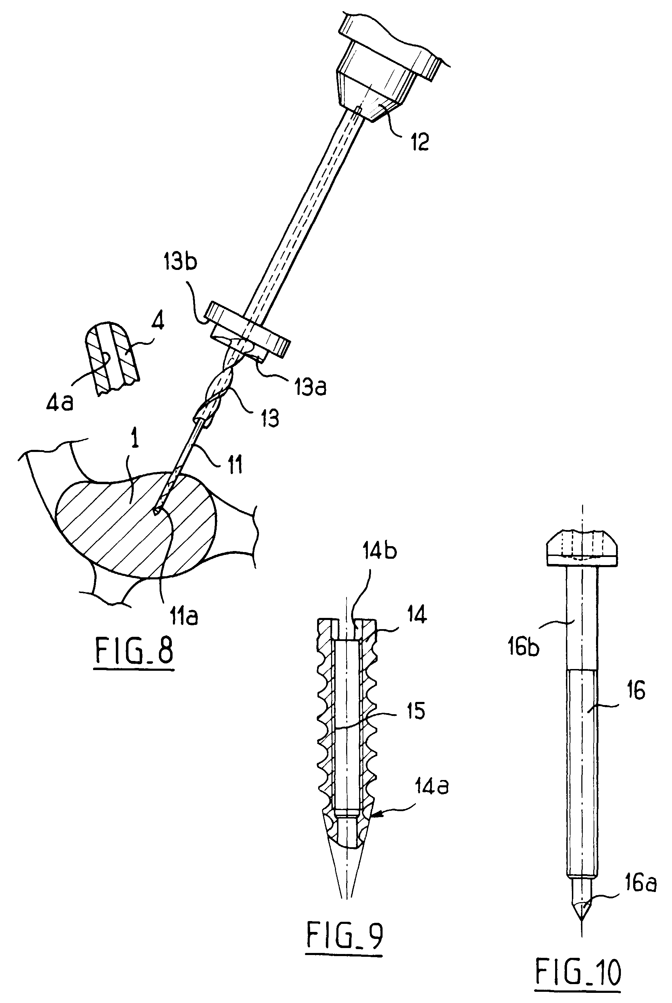

Ainsi donc, le chirurgien enfile le pointeau dans l'orifice 4a du greffon 4 et le présente sur l'os de l'omoplate 1. L'orientation et la position du pointeau lui permet d'ajuster précisément la position du greffon qu'il souhaite obtenir. Cette position étant fixée, le chirurgien impacte le pointeau pour faire pénétrer la pointe 8a du clou et la pointe 6a du corps tubulaire 6 à l'intérieur de l'omoplate. Cette impaction étant réalisée, le chirurgien dévisse le bouton 10 pour extraire le clou de la partie tubulaire du pointeau qu'il continue de maintenir en place. Cette partie tubulaire constitue alors, comme l'illustre la figure 7, un guide de perçage dans lequel on introduit une broche 11 dont l'extrémité 11a destinée à pénétrer dans l'os est formée comme un forêt. La mise en place de cette broche 11 est assurée au moyen d'un outil-moteur tournant 12. Lorsqu'on atteint la pénétration voulue de la broche dans l'os, on découple celle-ci de son organe moteur 12 d'entraînement et on retire la partie tubulaire du pointeau 5.

Afin de pouvoir retirer le greffon 4 de la broche, compte tenu de la mobilité réduite de ce greffon 4, on coupe la broche à une distance adéquate de son extrémité enfoncée dans l'os de manière que le greffon 4 puisse être extrait.

Comme illustré par la figure 8, la broche 11 ou du moins sa partie qui reste plantée dans l'os, constitue un guide de perçage pour une mèche 13 creuse qui est accouplée à l'organe d'entraînement 12 (ou un organe mieux adapté si nécessaire), voire qui est manoeuvré à la main à la manière d'une tarière. Cette mèche 13 permet donc de forer un alésage dans l'os 1 à un diamètre convenu et également d'aplanir au débouché de cet alésage une surface en forme de lamage autour de celui-ci grâce à un épaulement 13a de diamètre supérieur à celui de la mèche 13 qui forme une fraise à lamer, le diamètre de cette fraise à lamer étant déterminé à l'avance et correspondant approximativement au diamètre de la partie du greffon qui doit être rapporté sur l'os de l'omoplate. A ce propos on notera que cette partie greffon peut être elle-même usinée et dressée de manière à correspondre exactement au lamage réalisé au débouché de l'alésage réalisé dans l'os.

Le lamage réalisé par la fraise 13a lors de l'enfoncement de la mèche dans l'os 1 de l'omoplate est limité en profondeur par un épaulement 13b qui constitue une butée d'arrêt de la pénétration de la mèche 13 dans cet os.

Le diamètre extérieur de la mèche 13 correspond sensiblement au diamètre à fond de filet d'une cheville 14 autotaraudeuse telle que représentée en coupe à la figure 9. Cette cheville possède une extrémité inférieure 14a conique de sorte que cette partie constitue avec son filet extérieur une partie d'entrée de la cheville dans l'orifice foré par la mèche 13. Cette cheville 14 possède un alésage traversant axial 15 taraudé pour recevoir une vis telle que celle 16 représentée à la figure 10. A l'opposé de son extrémité conique, la cheville 14 possède une forme d'entraînement 14b grâce à laquelle, au moyen d'un tourne vis adapté à cette forme, on peut l'assujettir à l'intérieur de l'orifice foré dans l'omoplate. Son assujettissement sera tel que la partie supérieure de la vis au voisinage de sa forme d'entraînement 14b sera située légèrement en-dessous du fond du lamage qui entoure l'orifice accueillant cette cheville. Le chirurgien, pour achever l'opération, enfile la vis 16 dans le greffon 4 et procède au vissage de celle-ci dans la cheville 15. On aura noté que la vis 16 se termine par une extrémité 16a taillée en pointe pyramidale, ce qui permet de procéder à l'écartement des débris d'os qui pourraient avoir rempli l'alésage central 15 de la cheville 14. Elle possède également une partie 16b non filetée sous sa tête dont la longueur correspond à la longueur du greffon. Le greffon est ainsi serré entre la tête de la vis et le fond du lamage ménagé sur l'os autour du débouché de l'alésage, la force de serrage étant déterminée par le chirurgien. On notera donc que la tête de la vis 16 possède une embase de surface suffisante pour bien maintenir le greffon appliqué à l'os de l'omoplate. Cette tête présente également une empreinte apte à coopérer avec l'extrémité d'un tournevis adapté à la manoeuvre. Les empreintes de la cheville et de la vis seront de préférence différentes afin de constituer des détrompeurs pour les tournevis qui leur sont spécialement destinés.

## Revendications

1. Ensemble de moyens nécessaires à la réduction chirurgicale des instabilités de l'épaule selon la méthode LATARJET, par mise en place d'un greffon (4), comprenant un matériel ancillaire et un matériel implantable, caractérisé en ce que le matériel ancillaire comporte trois outils à utiliser consécutivement à savoir un pointeau (5), une broche (11), et une mèche (13), chaque outil formant un organe de guidage pour la mise en place de l'outil suivant, le pointeau formant organe de guidage du greffon (4) et en ce que le matériel implantable comporte une cheville (14) et une vis (16).

2. Ensemble selon la revendication 1, caractérisé en ce que le pointeau (5) comporte un manche (7) solidaire d'un corps tubulaire (6) et un clou (8) logé de manière amovible dans le corps (6), dont l'extrémité libre (8a) est en forme de pointe complémentaire de l'extrémité libre (6a) du corps tubulaire, et comportant des moyens (9, 10a) de sa liaison détachable avec le corps tubulaire.

3. Ensemble selon la revendication 2, caractérisé en ce que la broche (11) est formée d'une tige de longueur supérieure à celle du pointeau (5), de diamètre égal au diamètre intérieur du corps tubulaire (6), façonnée à l'une de ses extrémités (11a) à la manière d'un forêt.

4. Ensemble selon la revendication 3, caractérisé en ce que la mèche (13) est tubulaire, de diamètre intérieur égal au diamètre extérieur de la broche (11) et dont le diamètre extérieur est étagé (13, 13a).

5. Ensemble selon la revendication 4, caractérisé en ce que la longueur axiale de la section de la mèche (13a) de plus grand diamètre est déterminée par un épaule ment de butée (13b).

6. Ensemble selon la revendication 4 ou la revendication 5, caractérisé en ce que la cheville (14) est une cheville à visser autotaraudeuse dont le diamètre à fond de filet est sensiblement égal au diamètre de la mèche (13), le diamètre au sommet de filet étant décroissant vers l'extrémité sur une section (14a) de la cheville proche de cette extrémité pour former une section d'entrée de la cheville dans l'os et qui comporte un orifice axial (15) interne taraudé débouchant à chacune de ses extrémités.

7. Ensemble selon la revendication 6, caractérisé en ce que la vis (16) possède sous sa tête une partie (16b) non filetée de longueur égale à celle du greffon et, à l'opposé de sa tête une pointe pyramidale (16a) réalisée sur une partie non filetée de son extrémité.
